# EUROPEAN PATENT SPECIFICATION

(11) **EP 4 380 451 B1**
(45) Date of publication and mention of the grant of the patent: **20.05.2026**
(21) Application number: 22748415.1
(22) Date of filing: 27.07.2022
(51) Int. Cl.: A61B 5/282, A61B 5/318, A61B 5/01

(54) **DEVICE, SYSTEM AND METHOD FOR BIOMETRIC ELECTRODE TESTING**
VORRICHTUNG, SYSTEM UND VERFAHREN ZUR BIOMETRISCHEN ELEKTRODENPRÜFUNG
DISPOSITIF, SYSTÈME ET PROCÉDÉ DE TEST D'ÉLECTRODE BIOMÉTRIQUE

(30) Priority: 06.08.2021 GB 202111410
(43) Date of publication of application: 12.06.2024
(73) Proprietor: B-Secur Ltd, Belfast BT1 3FE (GB)
(72) Inventor: JARDINE, David, Belfast BT3 9DT (GB); BERMINGHAM, Alan, Belfast BT3 9DT (GB)
(74) Representative: Murgitroyd & Company
(86) International application number: PCT/GB2022/051973
(87) International publication number: WO 2023/012460

(56) References cited:
- WO-A1-2016/053731
- YUN-HSUAN CHEN ET AL: "Soft, Comfortable Polymer Dry Electrodes for High Quality ECG and EEG Recording", SENSORS, vol. 14, no. 12, 10 December 2014 (2014-12-10), pages 23758 - 23780, XP055498820, DOI: 10.3390/s141223758

## Description

### Field of Disclosure

This invention relates to a device, system and method for testing electrodes' interaction with human skin, in particular, the testing of dry electrode materials used in wearable devices for attaining biometric signals, such as electrocardiograph (ECG).

### Background

Wearable and biometric technology is becoming more evident and widely available on the world market, with increasing technologies and products being adapted into wearables and touch sensitive devices. One particular technological area of increasing interest is in the acquisition of biometric information through biometric sensing. Obtaining this information allows technology to enhance further as it can be implemented into a range of functional applications such as improved security (for example, personalised authentication) and personal health monitoring.

The acquiring and monitoring of biometric electrical signals may be of value in its own right, for example as a means of monitoring underlying physiological conditions or processes. Additionally, it may have value as a form of biometric sensing for other purposes such as for identification/ authentication. Biometric electrical signals, such as electrocardiograph (ECG), may be superior over other more common forms of personal physical detection, such as fingerprint recognition, iris response and voice activation. It is particularly favourable in security and authentication systems, as it is more difficult to replicate an individual's natural characteristics, such as their heartbeat.

In traditional ECG systems 'wet' electrodes are used to make a connection between the skin and front-end hardware. These electrodes are designed to ensure the best connection by adhering to a fixed position on the body and providing a conductive gel to reduce the contact impedance of the connection. These types of 'wet' electrodes are commonly used for clinical settings such as in hospitals where a patient is in a controlled setting. Historically ECG signals from patients have been recorded using electrodes that are typically made from silver/silver chloride material (Ag / AgCl) and an electrolytic gel material to act as a conductor between the skin and the electrode. ECG electrodes are transducers that allow charge, in the form of ionic charge from the body, to be converted into electrical current. This conversion is achieved through an electrochemical reaction between the user's skin, the conductive gel (electrolyte) and the electrode.

However, wet electrodes are not suitable for long term use for many reasons, primarily because the gel dries out over time leading to signal degradation over time. Further, the wet electrodes can cause skin irritation for certain users and, for each signal that is to be recorded, a minimum of two electrodes are required. Therefore, there are several reasons as to why wet electrodes are unsuitable for use in everyday non clinical biometric sensing applications such as ECG.

Given the rise in biometric acquisition systems incorporated in wearable devices and touch technological products, not just in a clinical setting, there is a need to use 'dry' electrodes instead of the common wet electrodes. Dry electrodes typically use a conductive surface in contact with the skin to generate a similar electrochemical reaction described for the wet electrode except there is no conductive gel (electrolyte) applied (hence the name dry electrode). The electrolyte is formed naturally by moisture/sweat/fluids on the surface of the skin. Dry electrodes have several advantages over wet electrodes, such as they can be used repeatedly with reduced degradation, only require contact or touch to the body (i.e. do not require a fixed position) and do not require a conductive gel. This makes them ideal for embedding in wearable and touch devices.

Nonetheless, using dry electrodes presents challenges to the biometric sensing system such as a less stable, higher impedance connection between the electrode and the skin and increased susceptibility to noise from sources such as body motion and mains electricity interference. Further, the electrochemical reaction generated between the conductive surface of the dry electrode and a user's skin can potentially add significant noise into the biometric electrical signal, such as ECG.

As such, finding suitable materials to act as dry electrodes for biometric sensing systems, in particular ECG applications, is difficult and one material that may be best for an application may not suit another. Therefore, there is a need to develop an efficient way to test dry electrodes, i.e. the materials used for such electrodes, to determine the noise influence of a particular electrode. Further the repeatability and accuracy of the various testing measurements needs to be maintained for the differing testing electrodes and skin locations as there are many characteristics of a dry electrode that impact the noise performance, and as such these characteristics need to be controlled.

Yun-Hsuan Chen et al., "Soft, Comfortable Polymer Dry Electrodes for High Quality ECG and EEG Recording", Sensors, volume 14, number 12, pages 23758-23780 (2014) discloses measuring electrode impedance on phantoms and human skin.

### Summary

There are many characteristics of a dry electrode that impact the noise performance. To determine the noise influence of an electrode these characteristics need to be controlled. Some of the characteristics include: electrode material and surface finish; coating and surface finish; surface material combinations (can be different from one electrode to another); surface area in contact with the skin; skin location for each electrode; pressure applied to electrode/skin contact; skin condition; user to user skin property variation; temperature; user movement; and electrical noise (mains/electronic noise from hardware). Also biometric electrical signals, such as ECG, are unique to every individual so it is difficult to deduce what is noise and what is the users ECG. Further, there are complexities involved in assessing the ECG signal quality and using this assessment to grade the electrode material in order to compare against multiple other options/materials. All of these aspects add complexity and risk to dry electrode material selection.

The following invention provides a method to test different electrode materials, for example dry electrode materials, and combinations while minimising/controlling the impacts on the noise performance as disclosed above. For example the proposed electrode test device, method and system may achieve this as follows: provides the ability to swap material plaques in and out of the device/system; provides the ability to test any combination of materials/coatings using test sample plaques; provides the ability to mask any shape or area the user requires; provides fixed electrode to user contact with fingertip and wrist electrodes; the device applies repeatable downward force to the user's finger(s) and the downward force applied to the wrist fixture is fixed so as to minimise motion artefact during testing; provides the ability to test a number of user's minimising skin property variations and conditions; electrode temperature can be monitored and displayed on screen; monitoring electrode offset potential; finger mounts and wrist electrodes are designed to reduce user movement; device has been validated to the recognised IEC60601-2-47 functional ECG testing standards and is wireless to remove mains interference; analytical software provides signal quality performance data which can be used to compare biometric performance, such as ECG, across multiple materials/combinations.

The present invention provides a device, system and method according to the independent claims.

As used herein, the term "limb or digit of the user" or like terms is used to describe a, and any, portion of the user's body.

The at least one user mount may comprise guides to hold the limb or digit of the user. The guides may provide fixed electrode to user contact minimising noise signal or motion artefacts from movement of user.

In some embodiments the electrode testing device comprises a biasing mechanism configured to bring the skin of a user's limb or digit into contact with the electrode to be tested; or to bring the electrode to be tested into contact with the skin of a user's limb or digit; or both. Advantageously this may ensure good and consistent contact between the skin of a user and the electrode. In some embodiments the at least one user mount comprises a biasing mechanism configured to bring the skin of a user's limb or digit into contact with the electrode to be tested; or to bring the electrode to be tested into contact with the skin of a user's limb or digit; or both. Advantageously this may ensure good and consistent contact between the skin of a user and the electrode.

In some embodiments the at least one electrode mounting holder comprises a biasing mechanism configured to bring the skin of a user's limb or digit into contact with the electrode to be tested; or to bring the electrode to be tested into contact with the skin of a user's limb or digit; or both. Advantageously this may ensure good and consistent contact between the skin of a user and the electrode.

In some embodiments the biasing mechanism comprises variable tension pins.

In some embodiments the electrode testing device comprises variable tension pins configured to bring the skin of a user's limb or digit into contact with the electrode to be tested; or to bring the electrode to be tested into contact with the skin of a user's limb or digit; or both.

The at least one user mount may comprise variable tension pins. The variable tension pins may ensure an equal contact pressure is maintained between a user's limb or digit and the electrode. Further, it ensures the same tension can be set across all of the user mounts when subject to testing. Advantageously tension pins may be marked indicating a tension value or pressure and therefore aid repeatability of consistent tension value or pressure.

In some embodiments there comprises at least one variable tension pin. In some embodiments there comprises at least two variable tension pins. In some embodiments there comprises at least 4 variable tension pins. Depending on the location size and general configuration the larger the number of variable tension pins may give a more consistent and repeatable tension or pressure of contact between the electrode to be tested and the skin of a user. The tension pins support a tension plate, thus uniform tension is achieved by setting all of the tension pins to the same height or tension value. Advantageously having 4 variable tension pins is a good compromise of giving a good repeatable tension between the electrode to be tested and the skin of the user, and size and complexity of the device. Further, the variable tension pins allow for any finger size of a user to be inserted into the electrode mounting holder for test as the height of the tension plate is adjustable via the tension pins.

In some embodiments at least one variable tension pin is marked to indicate a tension. In some embodiments at least one variable tension pin is marked to indicate a tension or pressure asserted on the limb or digit of the user, or electrode, biasing the electrode or limb or digit of the user towards each other.

At least one electrode may be a dry material. In some embodiments at least one electrode comprises of a dry electrode.

The electrode mounting holders may be detachable from the electrode testing device. At least one user mount may be detachable from the electrode testing device. Having detachable electrode mounting holders and user mounts provides easy transportation of the device, adds to the compact size of the device and allows other different electrode types to be connected to the device.

At least one electrode mounting holder comprises: a plurality of electrode material sample holders; and an electrode material mounting slot, wherein an electrode material sample holder of the plurality of electrode material sample holders is be housed within the electrode material mounting slot. The use of multiple sample holders, which are housed within the electrode material mounting slot provides the ability to swap material plaques in and out of the device with ease, allowing multiple electrodes to be tested in short succession.

The plurality of electrode material sample holders may comprise a plurality of material contact areas. Variations in contact areas gives information on the quality of signal for a particular area, and as such allows electrodes to be tested that may be constrained by size depending on design or application.

The at least one user mount may be replaced with a strap-type electrode suitable for strapping to a user's body. Having strap-type electrodes increases the number of locations on a user's body from which a biometric electrical signal can be obtained, widening the scope of dry electrode applications.

The variable tension pins may be configured to provide matching incremental tension values on the one or more user mounts. The incremental values improves the accuracy of the biometric signal when using more than one user mount as the user mounts may be set with the same tension.

The electrode testing device may further comprise a power source. In some embodiments the power source may be a battery. Advantageously the battery aids with transportation and removes unwanted A/C noise from the signal.

The electrode testing device may further comprise a display screen. In some embodiments the display screen is an LCD screen.

The display screen may display information related to detection status, connection status, signal quality, biometric electrical signal values, electrode temperature, and/or battery life. Having a display screen on the device provides instant information to the user on the functions of the device without the need to use the analytical software.

The electrode testing device may further comprise of a wireless network communication module. Again the wireless function aids in removing noise interference indicative of wired connections.

The biometric electrical signals may be uploaded to a cloud and analysed remotely using biometric analysis software. The software provides signal quality performance data which can be used to compare biometric signal performance across multiple electrode materials and combinations. Having a cloud server allows the data from the electrode testing device to be analysed remotely and to enable detailed analytics of the data.

In some embodiments the electrode testing device further comprises a wet electrode connection input. Advantageously this may allow comparison of a dry electrode to a wet electrode. The wet electrode used with the present invention may be a Right-Leg-Drive (RLD).

In an aspect of the invention there is provided an electrode testing method comprising testing an electrode using the electrode testing device as described herein.

The biometric electrical signal may be an electrocardiograph (ECG) signal.

The method may further comprise acquiring offset potential data of each electrode interface. Acquiring the offset potential provides a measure of the noise and thus the performance of each individual electrode adding to the overall analysis and quality assessment of each test electrode material.

Any one or more of the features of any aspect, embodiment or example as described herein may be combined with any one or more of any other feature of any aspect, embodiment or example described herein.

### Brief Description of Drawings

By way of example the invention will be described with reference to the figures in relation to particular non-limiting examples.
Fig.1 shows a diagram of an electrode testing device according to the present invention.
Fig. 2A diagram represents a close up of one of the electrode mounting holders of the electrode testing device with details of the user mounts, variable tension pins, electrode material mounting slot and the interchangeable material sample holders.
Fig. 2B depicts the variable tension pins of a user mount, highlighting the variable adjustments of the tension pins into the pre-defined holes.
Fig. 3 is a diagram of the electrode testing device according to another aspect of the invention with a strap-type electrode in place of one of the electrode mounting holders.
Fig. 4 is a diagram of an example strap-type electrode, the example here being a wrist strap.
Fig. 5 is diagram illustrating the electrode testing system, including a user, the electrode testing device, a server and a computing device.
Fig. 6 is a flow diagram of the method steps for acquiring a biometric electrical signal using the electrode testing device.
Fig. 7A is a flow diagram of the example method steps of an electrode material baseline test using the electrode testing device.
Fig. 7B is a flow diagram of the example method steps of an electrode contact area test using the electrode testing device.
Fig. 7C is a flow diagram of the example method steps of an electrode material ageing test using the electrode testing device.
Fig. 7D is a flow diagram of the example method steps of an electrode contact time test using the electrode testing device.

### Detailed Description

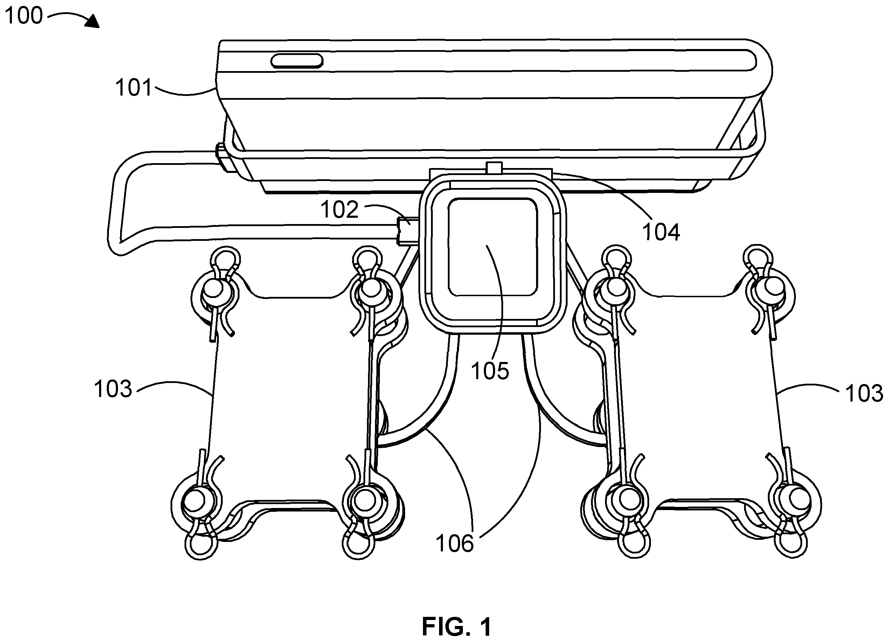

Fig. 1 depicts a simple diagram of an electrode testing device 100 as described throughout this document as a preferred embodiment. Fig. 1 illustrates the electrode testing device 100 comprising the various components required to test electrode materials according to the preferred embodiment. As shown in Fig. 1 the device 100 comprises a power source 101. Any common forms of power sources may be implemented or connected to the device, however, it is preferable that the source is a battery, enabling portability of the electrode testing device. The portability of the electrode testing device is further enhance by the table top sized dimensions of the device and by the number of components that are detachable from the main body 102. Typical height, width, depth dimensions are in the order of 100 mm - 150 mm. An example electrode testing device 100 dimensions are approximately height = 100 mm, width = 150 mm and depth = 120 mm. It will be realised that the overall electrode testing device 100 dimensions may change depending on which components are present within the electrode testing device 100. These component features also allow different types of testing to occur to gain various electrode characteristics. The different types of testing and measurements will be discussed later in the document.

The main body 102 of the electrode testing device 100 contains the electronic hardware for operating the electrode testing device. The hardware comprises one or more electrical signal acquisition modules; an ECG signal conditioning integrated circuit; a power management module, a microcontroller, non-volatile memory (NVM), a wireless network communication module; and protection circuitry to protect the electronics and the user. The main body 102 also comprises power and mode buttons for switching the device on and off and switching between different modes. The mode button allows the user to switch between acquisition and viewing modes on the electrode testing device 100.

In this example the electrode testing device 100 also comprises a display screen 105 located on the main body 102, providing information to the user on various functions of the device and test measurements. The information displayed may include, detection status, connection status, signal quality, biometric electrical signal values, battery life, electrode temperature and other data. For example, to indicate that the leads have been detected, or that the device has successfully connected to a remote device, via Bluetooth, WIFI or the like, or provide on the spot Heart Rate values or for monitoring the temperature at the electrodes. The display screen 105 may be an LCD, OLED, LED screen, or the like. The display screen 105 may also be touch screen enabled. Different types of data can be displayed using a mode button for changing the display mode of the display screen 105.

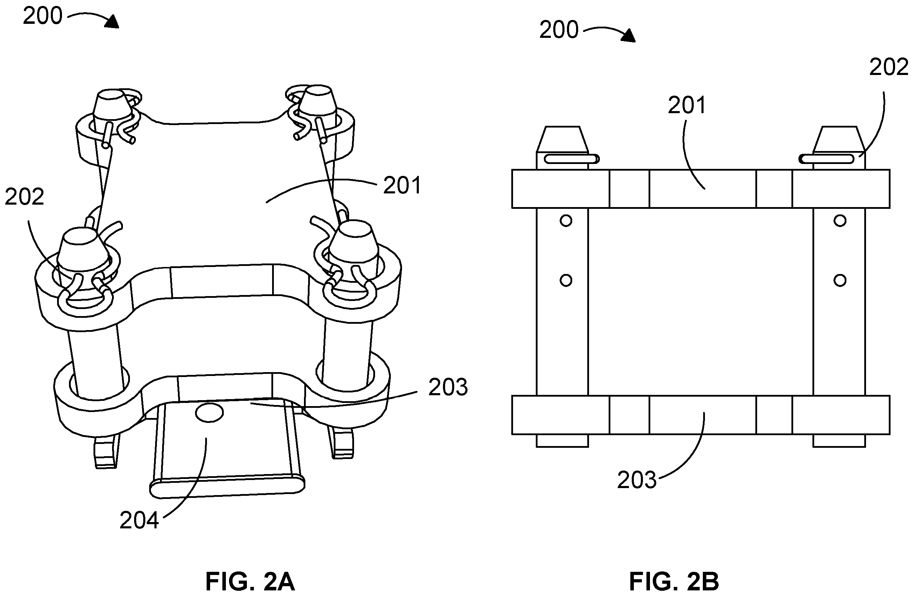

Fig. 1 also illustrates the electrode mounting holders 103. These can be detachable from the main body 102 of the device for ease of electrode test material loading, and to exchange with a strap-type electrode strap suitable for strapping to a user's body, such as a wrist electrode or chest electrode strap, or any other body contact type electrode. Details of the electrode mounting holders 103 will be discussed with reference to Fig. 2. Further, although the configuration of the electrode testing device 100 in Fig. 1 depicts two electrode mounting holders 103, it will be realised that the electrode testing device 100 may comprise one or more electrode mounting holders 103 depending on the application.

The electrode testing device 100 also comprises a wet electrode connection input 104, allowing a wet electrode to be connected to the device. The wet electrode may be a Right-Leg-Drive (RLD) electrode and is connected to the electrode testing device 100, via a cable, during testing of dry electrodes. Using a wet electrode connection between the electrode testing device 100 and the user, provides a reference biometric electrical signal to be obtained from the user. A fresh wet electrode is used for each user when conducting various measurements in testing of dry electrode materials in order to achieve continuity and consistency in the electrochemical reaction.

Figs. 2A and 2B represent a close up of one of the detachable electrode mounting holders 103. The diagram 200 in Fig. 2A highlights the different components of the electrode mounting holders 103 and the features required to obtain electrode testing data from a finger contact of a user. The electrode mounting holder comprises a user mount 201 for receiving a user's limb or digit, such as a finger. The user mount 201 is configured in such a way that a user can place a limb or digit in the mount, preferably a finger, and once the finger is inserted the finger is limited in lateral movement by the user mount guides. The finger is further held in place by a tension plate with variable tension pins 202, as shown in Fig. 2A. One or more tension pins 202 can be used to secure the finger within the mount and limit vertical movement. The tension pins 202 also provide control over the contact pressure of the user's finger to the electrode, such that the same contact pressure can be used across various tests (if such variable is to be kept constant). The tension pins 202 can be varied such that the top part of the finger mount, i.e. the tension plate, can be held in different vertical positions, as shown in Fig. 2B. The tension pins allow the downward pressure to be fine-tuned on spring contacts that are built into the tension plate of the finger mount. These spring contacts provide a range for each of the height settings. The tension pins 202 are moved into pre-set holes in a vertical spindle to achieve a desired height for the tension plate of the user mount 201. The pre-set holes are identical on each spindle to allow the one or more tension pins 202 to reside at the same height. Thus, the variable tension pins 202 allow different contact pressures to be selected, depending on the testing requirements of the electrode. For example, tension pins 202 placed in pre-set holes closer to the bottom surface of the user mount 201 will provide increase contact pressure between the user's finger and the electrode than tensions pins 202 placed further away from the bottom surface of the user mount 201. In the diagrams of Figs. 2A and 2B there are four tension pins 202 shown, located in the corners of the user mount 201 and tension plate, which aids in providing a uniform contact pressure across the user mount 201. However, it will be realised that the electrode mounting holder 103 may be configured to have any number of tension pins 202 and of any size, depending on the application and the body part to be in contact with the electrode.

The electrode mounting holder 103 of Fig. 2A also comprises an electrode material mounting slot 203 and a plurality of interchangeable material sample holders 204. The mounting slot 203, in the example in Figs. 2A and 2B, is located in the base of the electrode mounting holder 103. Each of the different electrode materials that are put under test are placed in one of the material sample holders 204 and inserted into the electrode material mounting slot 203. On insertion of the material into the slot, an electrical connection between the test electrode material and power supply 101 is made, via the electrode mounting holder 103, a connecting cable 106 and the main body 102 of the device (see Fig. 1). The material sample holders 204 comprise different sized apertures allowing the contact area between the electrode material and the user to be altered. For example, the apertures contact sizes of the material sample holders 204 may be chosen from 3 mm to 12 mm in diameter. If more than one electrode mounting holder 103 is used in a given test, i.e. two simultaneously, the same material sample holder 204 contact size must be chosen. When only one electrode mounting holder 103 is used the other point of contact may be a strap-type electrode which is discussed below. Mismatching electrode material contact area sizes in the electrode mounting holders 103 will adversely impact the test results. Also, if more than one electrode mounting holder 103 is used in a given test or measurement, the same test electrode material should be inserted into the chosen material sample holders 204. Again, mismatching materials will not provide accurate results.

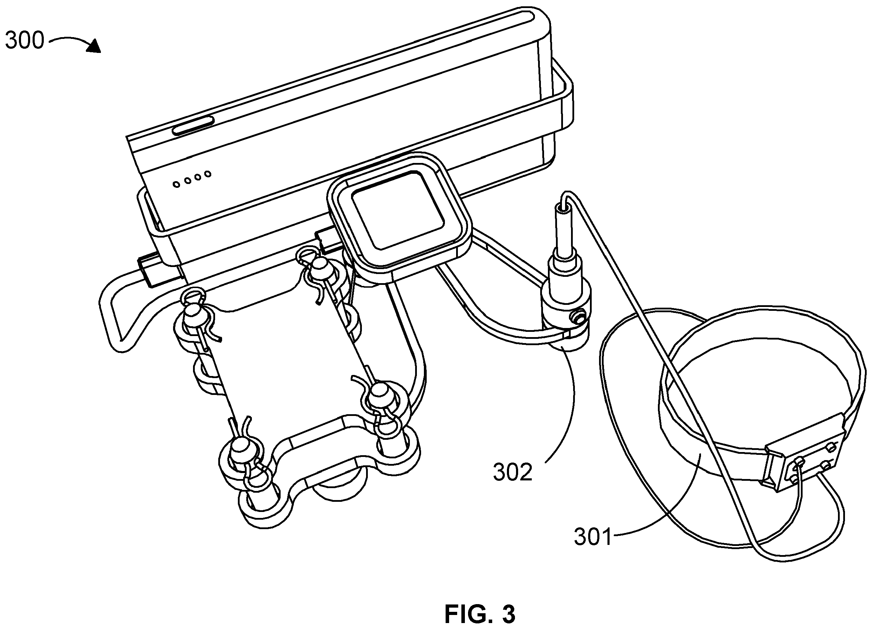

Fig. 3 illustrates an alternative arrangement 300 of the electrode testing device 100, whereby one of the at least one electrode mounting holders 103 is replaced with a strap-type electrode 301. The strap-type electrode 301 allows biometric electrical signal measurements to be taken from elsewhere on the body. In the present case the strap-type electrode is a wrist strap electrode, such that it mimics a watch-like wearable device. It will be realised that other strap-type electrodes may be used to obtain biometric electrical measurements from other areas of the body, for example another limb strap electrode, a chest strap electrode or the like. As shown in Fig. 3, the strap-type electrode 301 is easily connected into the port 302 of the electrode mounting holder 103.

Further, the electrode testing device may also be arranged such that the offset potential of the electrodes may be measured. Due to differences in the skin, electrode material, moisture on the surface of the skin, etc., the offset potential is an important parameter to measure as it provides information as to how well the system is balanced over the two electrodes. The ECG signal alone would not provide this information. The offset potential is of great importance when users wish to have different electrode materials and/or electrode coatings for each electrode, i.e. left electrode and right electrode, or wish to place each of the electrodes on different body parts, i.e. finger and wrist. Each electrode skin interface generates an electrochemical reaction. For dry electrode materials the electrolyte driving the electrochemical reaction is formed naturally by moisture/sweat/fluids on the surface of the skin, unlike wet electrodes which use a conductive gel. Nevertheless, if there was a balanced system where all factors were controlled one electrode may produce +0.5 V and the other electrode produce -0.5 V in the respective electrochemical reactions, thus the offset potential would be 0 V. However, this may not be the case when altering electrode material or placement of electrode contact on a user's skin. As such, a large offset potential could be generated, for example >1 V, which may exceed the hardware input voltage range depending on the application for the electrodes. The electrode testing device 100 has the capability to measure such offset potential between each electrode by measuring the separate electrochemical reactions. The electrode testing device 100 can perform both electrochemical reaction measurements simultaneously and provide the user with the voltage generated at each electrode, as well as the overall offset potential voltage. The main body 102 of the electrode testing device 100 contains the appropriate electronic hardware for measuring the offset potential using the electrode testing device 100. See above for hardware components.

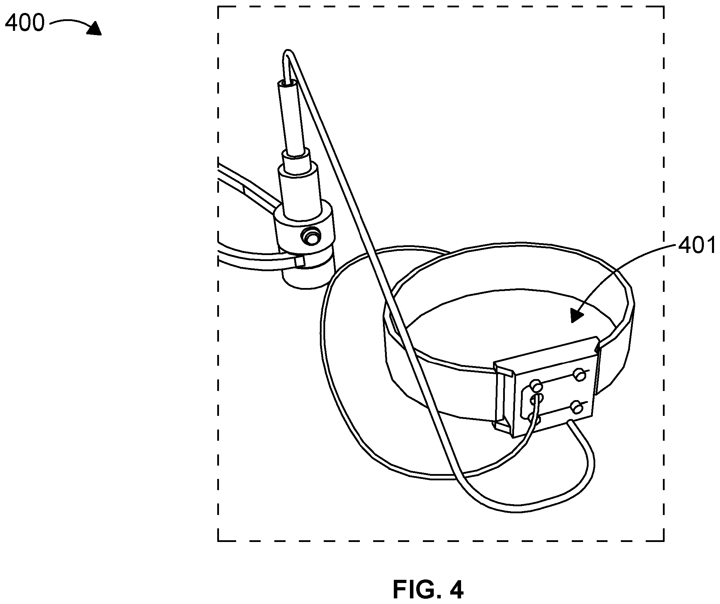

Fig. 4 is a more detailed view 400 of the strap-type electrode 301, in this case a wrist strap electrode, with electrodes positioned on the strap to take biometric electrical measurements, such as ECG. The electrode 401 shown in the example wrist strap electrode 400 in Fig. 4 is positioned on the inside of the wrist strap electrode such that the electrode makes contact with a user's wrist when the wrist strap electrode 400 is worn by a user. The wrist strap electrode 400 may be used in conjunction with the electrode mounting holder 103, where contact is made with the inside electrode 401 of the wrist strap and from the user mount 201 of the electrode mounting holder 103. The material used for the electrodes 401of the wrist strap electrode 400 may be a commonly used material, such as 316L Stainless Steel, when testing the material in the sample mounting holder 204 of the electrode mounting holder 103. For other measurements the electrode material of the wrist strap electrodes 401 may be a test material that matches the material in the electrode mounting holder 103.

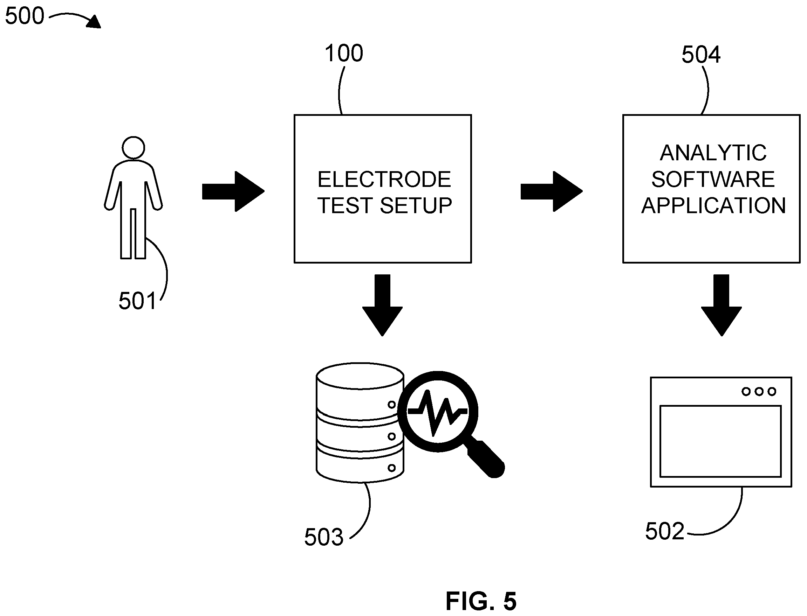

The electrode testing device 100 is part of an electrode testing system 500 as shown in the diagram in Fig. 5. The system 500 comprises a user or test subject 501, the electrode testing device 100, a remote database of biometric data 503, a computing device 502 and a cloud server 503 and biometric analytic software 504 which is executed using an application between the computing device 502 and cloud server 503. The electrode testing system 500 provides live feedback on the electrode test results, and does not require any unnecessary data manipulation or further analysis in order to determine if a candidate electrode material is suitable for the chosen application. The electrode testing system 500 also provide a report at the end of the data capture.

As discussed earlier, the electrode testing device 100 can present certain information on the display screen 105, such as battery life, connection status, heart rate, etc. However, for full biometric analysis it is preferable that the electrode testing device 100 is connected to a remote computing device 502, such as a PC, tablet, mobile phone, etc., that is running the analytic software application. The connection between the devices is wireless to remove any unwanted noise associated with line and A/C connections. The analytic software application 504 provides the connection and retrieval of biometric signal data, such as ECG, from the electrode testing device 100 and outputs the analysis of the signal on the computing device 502 interface in real time. The application can also work by connecting to the remote cloud server and relaying the biometric data, i.e. ECG, over the internet connection. The cloud server analyses the ECG signal and responds to the application with the outputs such as heart rate, number of heartbeats detected etc. Other common outputs from the application may be provided to the user to assess the signal quality of the ECG signal recorded. The application also displays system test information to ensure the system is operating correctly.

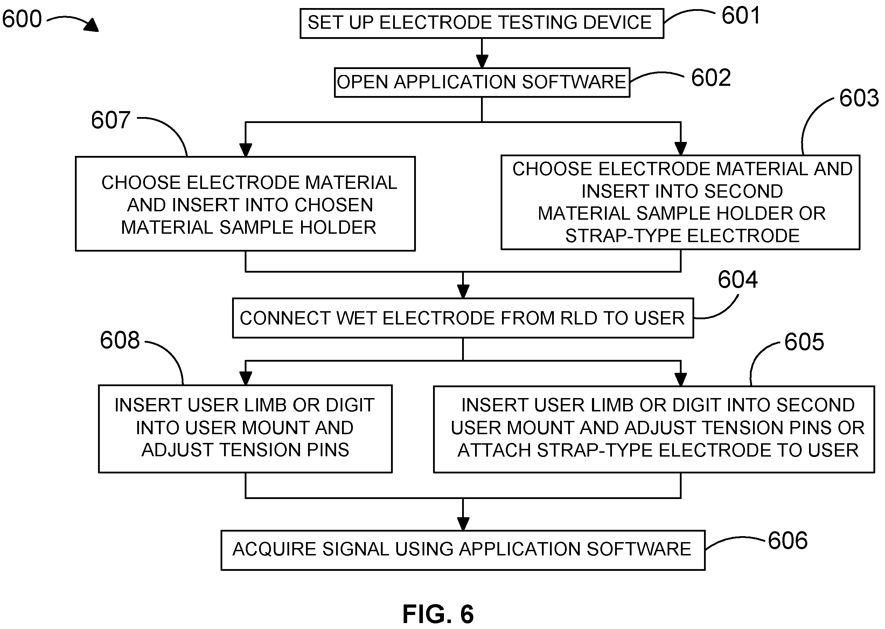

Fig. 6 is a flow diagram 600 depicting the method steps, of an embodiment of the present invention, for acquiring a biometric electrical signal, such as an ECG. The first step 601 is to set up the electrode testing device 100 by connecting all the necessary components for acquiring a biometric electrical signal. For example, the battery 101, electrode mounting holders 103 and/or strap-type electrode 301, and wet electrode connection (RLD) 104 are all connected to the main body 105 of the device. The application software 504 is opened 602 and the electrode testing device 100 powered on. A confirmation of a connection between the device 100 and software 504 is obtained. The next step is to set up the sample materials 603/607 in the material sample holders 204 of the electrode mounting holders 103 (or strap-type electrode 301), making sure the same test or reference material and the same sample holder 204 contact size is used across the electrodes. The tension pins 202 are then set so the tension plate resides at a desired level, again making sure the contact pressure is the same across the user mount 201 electrodes and the user's finger is inserted into the user mount 201, as in step 608 of Fig. 6. The second finger is inserted into the user mount 201 or the strap-type electrode 301 is attached 605. The wet electrode 104 is also connected to the user 604. From the software application 504, signal acquisition is initiated 606 on the user being connected to the electrode testing device 100. This may be automatic or may require human input. The signal will be continuously recorded until the acquisition is stopped via the application 504. The acquired signal will then be stored locally on the computing device 502 or can be stored in the cloud server 503 or a remote database 501, and the measurement can be repeated if needed or the system set up for another test.

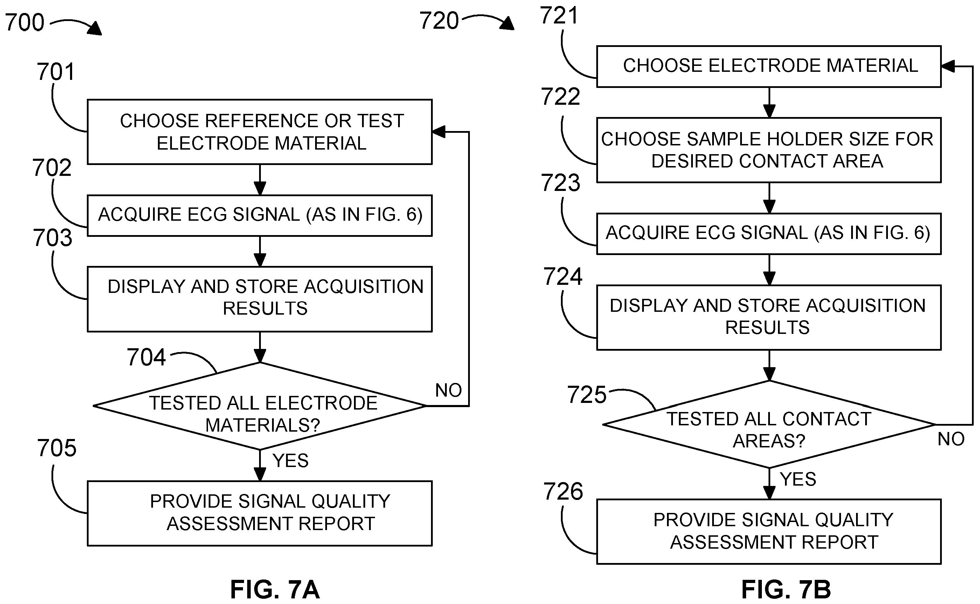

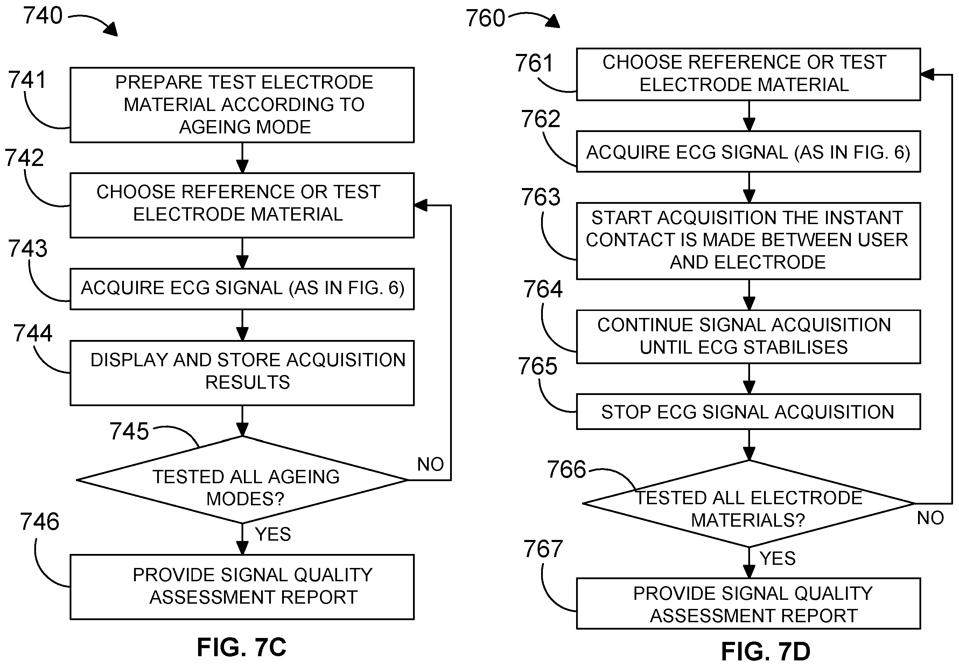

In order to narrow down the number of electrode testing materials required to be tested a baseline test is performed, as shown in the flow diagram 700 of Fig. 7A. This baseline test reduces extensive electrode material testing and has the advantage that it saves time and cost. The following method is directed towards ECG measurements and ECG test material electrodes, but it will be apparent that other biometric type measurements and electrodes may be used. A reference ECG electrode material is chosen 701 to acquire an ECG signal from the electrode testing device 100, for a particular user in a particular environment (i.e. the external factors are fixed). A common high performance material is used as a reference as the characteristics of such an electrode are well known, thus allowing a fair comparison of the data with the other test electrode materials.

The user acquires the reference ECG signal 702 as set out in the flow diagram 600 of Fig. 6, and the results are displayed and stored locally 703 in the application software of the electrode testing system 500 for instant review. As mentioned previously, the data and/or results may be exported or transmitted to a cloud server or storage system or the like. Once the reference ECG signal is acquired the various electrode test materials may be acquired under the same conditions. The reference material is replaced by a test material 701 and the ECG signal is acquired 702. Again, the acquisition method steps follow those in flow diagram 600 of Fig. 6 and the results are displayed and stored locally 703 in the application software. The acquiring of the ECG signal is repeated for each electrode test material under test 704. All electrode test materials results can be viewed alongside the reference material results to make a quick comparison of the suitability of each electrode test material as an ECG electrode. The comparison of various parameters of the electrode test materials may be provided in a signal quality assessment report 705 generated by the analytic software in order for the user to make a quick assessment. From the data the poor performing test materials can be discarded and the best performing test materials can be subject to more in depth testing, thus reducing the number of materials being subject to in depth testing. Performing the baseline tests on numerous user's or test subjects that represent a wide range of skin types (age, skin type, gender, etc) will ensure greater reliability on the test results. The greater the subject pool, the greater the population representation.

An important parameter to consider when testing electrode materials is the size of the contact surface area of the electrode with a user's body. Thus, evaluating the test electrode material performance while varying the contact surface area is of great importance, especially when the size of the electrode may be restricted when incorporated into a particular touch technological product or wearable device. A flow diagram 720 of the method steps is provided in Fig. 7B. As discussed earlier in this document, material sample holders 204 of varying aperture sizes may be chosen 722 and can be used to achieve this. The ability to quickly exchange the material sample holders 204 in the electrode testing device 100, while maintaining continuity of the other variables, reduces the overall measurement time considerably, especially when having to measure several test materials. Thus the electrode testing device 100 gives quick and accurate results 726 without the need for extensive laboratory equipment.

Reducing the aperture size restricts the contact area between the test material and the body of any test subject or user. Therefore, for a selected test electrode material 721 the sample holder 722 is changed for each ECG acquisition measurement 723 in incremental aperture size and the results are provided in the application software 724. This is repeated until all the required contact areas have been test 725. Similar to the baseline test, assessment can be made based on the signal quality assessment report 726. The electrode testing device 100 in combination with the various material sample holders 204 allows testing of any surface up to 200 mm². The surface area may be altered in increments of 10 mm², allowing the point of failure in contact area size to be found for the required application. Again, performing the contact area tests 720 repeatedly on more user's or test subjects will ensure greater reliability on the test results, thus increasing the accuracy of the chosen contact surface area.

Another important characteristic to consider when selecting an appropriate electrode material for a particular biometric sensing purpose is how well the selected material ages. In particular, the longevity of the performance of the electrode when monitoring biometric electrical signals, such as ECG. For example, after some time the electrode may become scuffed, scratched, exposed to certain chemicals or be subject to other stresses which may alter the material properties of the electrodes. Especially if the electrode is on the outer parts of the device. A change in these properties may affect the biometric, i.e. ECG, monitoring performance. Thus, the ageing performance of the test electrodes should be considered to see if the electrodes are still sufficient after a period of time. Further, the ageing test can also aid in providing particular limitations or guidelines to adopt in certain electrode applications.

In relation to the ageing effects coatings may be applied to the electrode surface to protect the electrode from age related damage as mentioned above. Particular coatings may be used for other purposes, such as increasing conductivity. Thus, the coating applied to an electrode material can also be tested for investigating other characteristics of the coatings. As conductive coatings come in different compositions, thicknesses, finishes, as well as undergoing different curing processes, the electrode testing device 100 is able to make comparisons between the different coatings not just the electrode material.

In order to perform the ageing test on test electrode materials, as disclosed in the flow diagram 740 of Fig, 7C, the selected test electrode material must be prepared for a particular ageing mode 741. For example, a sample of the material is scratched, subjected to abrasion, salt fog, salt water, corrosive gas or liquid, and/or any other modes that will impact the ageing of the electrode. The ageing modes cause degradation in the electrode material which reduces the biometric sensing of the particular application, such as ECG. As with other tests described in this document, a reference measurement is made with a sample electrode material 742 which has no ageing effects imparted on it. It is important to keep the controllable variables the same throughout the testing, such as user, contact area size, temperature, etc. The acquisition of the electrical signal 743 is taken as described according to Fig. 6. Following the acquisition of the reference signal, each of the "aged" electrode material samples 742 are inserted into the chosen sample holder and a signal is acquired 743 for each. Again the results from the biometric electrical signal acquisitions, such as ECG, are displayed in the application software 744, with the reference results displayed alongside the various "aged" electrode material sample results. As with the other tests, a signal quality assessment report is provided 746 of the ageing test results. Any material that withstands the various ageing modes so as not to reduce ECG performance can be considered with confidence to maintain performance for the lifetime of the application. Thus, selecting an electrode material for an application which requires the electrode to be in-situ for a long period, is easily selected using the electrode testing device 100 and this aforementioned method. Further, this method is useful when testing multi-layered structure electrode materials or a material which has had a coating applied on the surface, i.e. does the electrode still perform adequately when the first layer of the multi-layered structure or the coating has been scratched.

A further aspect that can influence the acquisition performance of biometric electrical signals using dry electrodes is the contact time between the electrodes and a user or subject's body. For example, in the watch-like strap electrode 400 as shown in Fig. 4, the time for a signal to be gained when a user touches the outer electrode surface 402 with their finger. Electrodes used for ECG acquisition, often improve in performance with an increase in contact time between the electrodes and the user's body. For example, when the watch-like strap electrode has been worn by the user for a few minutes when compared to when the user initially puts the device on. This test will aid in providing an end user of the wearable product with a waiting time after putting on the device before trying to record ECG.

The contact time test, as shown in the flow diagram 760 of Fig. 7D, is executed by starting a biometric electrical signal acquisition 762 of a chosen electrode material 761 using the electrode testing device 100 the instant contact is made between the user and the test electrodes 763. The biometric electrical signal acquisition, in this case ECG, is continuously recorded 764 until the point at which the performance level of the ECG stabilises, i.e. remains constant and does not improve any further. After a suitable time, and at which the ECG performance levels, the recording is stopped 765. This is repeated for all test electrode materials 766. For improved ECG performance time analysis and for comparison with other materials, the ECG signal could be measured within sequential time windows. Therefore, the quality of the signal can be compared between each time window for a given test electrode material and compared with the same time windows recorded for other test electrode materials. For example, if a recording is made for 5 minutes and broken into five 1 minute long windows, the performance could be compared between each 1 minute time windows for a given material. Similarly, the same minute window of one material can be compared with the same minute window of another material. The contact time test results, including selected time windows, is provided in a signal quality assessment report 767 for test electrode material comparison. Recording such data across multiple test subjects or users will help establish what length of contact time is necessary for a particular test electrode material in order to ensure the ECG recordings are of acceptable quality for such test electrode material.

A further test measurement that is of importance in electrode applications, and can be conducted by using the electrode testing device 100, is the offset potential. Offset potential measures the potentials generated at each electrode interface. The electrode testing device 100 has the capability to measure the offset potentials of each electrode independently, wherein the measured voltage is unique to each electrode. As such, this offset potential measurement allows the noise and performance of mixed electrode materials to be analysed. For example, if there was a need to mix 2 or more materials for a particular application, then the offset potential measurement using the electrode testing device 100 could determine the performance of each. In addition, the offset potential measurement also provides the necessary data to identify if the AFE maximum offset potential requirements are met for a particular electrode material.

For all of the aforementioned testing methods, it is important to control as many variables as possible to provide accurate results and reduce any external factors which may influence the test results. These include but are not limited to: movement of a test user to ensure repeatability of results and avoid unwanted motion artefacts; sweat build up from over touching electrodes during test; use of hand creams, sanitisers or other chemicals on a user's skin; temperature and humidity of the test environment; wearing of gloves, watches or clothing that may cause change in temperature or sweat content on the skin surface between measurements.

## Claims

1. An electrode testing device (100), for testing biometric sensing electrodes, the electrode testing device (100) comprising:
at least one electrode mounting holder (103), for holding an electrode to be tested, wherein the at least one electrode mounting holder (103) comprises an electrode material mounting slot (203), a plurality of electrode material sample holders and at least one user mount (201) for holding a limb or digit of a user, wherein an electrode material sample holder (204) of the plurality of electrode material sample holders is housed within the electrode material mounting slot (203), wherein the electrode to be tested is placed in the electrode material sample holder of the plurality of electrode material sample holders, and wherein the at least one user mount (201) is configured to enable the electrode to be in contact with the skin of the user; and
an electrical signal acquisition module, wherein the electrical signal acquisition module acquires biometric electrical signals from the user through the contact of the electrode with the skin of the user.

2. An electrode testing device (100) of claim 1 wherein the at least one user mount (201) comprises guides to hold of the limb or digit of the user.

3. An electrode testing device (100) of claim 1 or claim 2 wherein the at least one user mount (201) comprises variable tension pins (202), optionally wherein the variable tension pins (202) are configured to provide matching incremental tension values on at least one user mount (201).

4. The electrode testing device (100) of any preceding claim, wherein the at least one electrode is a dry material.

5. The electrode testing device (100) of any preceding claim, wherein the electrode mounting holders (103) are detachable from the electrode testing device (100), optionally wherein the plurality of electrode material sample holders comprise a plurality of material contact areas.

6. The electrode testing device (100) of any preceding claim, wherein the at least one user mount (201) is detachable from the electrode testing device (100), optionally wherein one of the at least one of the user mounts (201) is replaced with a strap-type electrode suitable for strapping to a user's body.

7. The electrode testing device (100) of any preceding claim, further comprising a power source (101), wherein the power source (101) is a battery.

8. The electrode testing device (100) of any preceding claim, further comprising a display screen (105), wherein the display screen (105) is an LCD screen, optionally wherein the display screen (105) displays information related to detection status, connection status, signal quality, biometric electrical signal values, electrode temperature, and/or battery life.

9. The electrode testing device (100) of any preceding claim, further comprising a wireless network communication module.

10. The electrode testing device (100) of any of claims 1 to 9, wherein the biometric electrical signals are configured to enable uploading to a cloud and analysed remotely using biometric analysis software.

11. An electrode testing method of the electrode testing device (100) according to any preceding claim, the electrode testing method comprising:
installing one or more electrodes into the at least one electrode mounting holder (103);
placing a limb or digit of a user into the at least one user mount (201); and
acquiring, from the electrical signal acquisition module, a biometric electrical signal from the user.

12. The electrode testing method of claim 11, wherein the biometric electrical signal is an electrocardiograph, ECG, signal.

13. The electrode testing method of claim 11, further comprising acquiring offset potential data of each electrode interface.

14. A system (500) for testing electrodes, comprising:
an electrode testing device (100) according to any of claims 1-10;
an electronic computing device (502) comprising biometric analysis software (504);
a wireless communication system operable to effect communication of data between the electrode testing device (100) and the electronic computing device (502); and
a cloud server (503).

15. The system (500) of claim 14, further comprising a digital temperature meter.

## Patentansprüche

1. Eine Elektrodentestvorrichtung (100) zum Testen von Elektroden zur biometrischen Messung, wobei die Elektrodentestvorrichtung (100) Folgendes beinhaltet:
mindestens einen Elektrodenbefestigungshalter (103) zum Halten einer zu testenden Elektrode, wobei der mindestens eine Elektrodenbefestigungshalter (103) einen Elektrodenmaterialbefestigungsschlitz (203), eine Vielzahl von Elektrodenmaterialprobenhaltern und mindestens eine Benutzerbefestigung (201) zum Halten einer Gliedmaße oder eines Fingers eines Benutzers beinhaltet, wobei ein Elektrodenmaterialprobenhalter (204) der Vielzahl von Elektrodenmaterialprobenhaltern innerhalb des Elektrodenmaterialbefestigungsschlitzes (203) untergebracht ist, wobei die zu testende Elektrode in dem Elektrodenmaterialprobenhalter der Vielzahl von Elektrodenmaterialprobenhaltern platziert ist und wobei die mindestens eine Benutzerbefestigung (201) konfiguriert ist, um zu ermöglichen, dass die Elektrode in Kontakt mit der Haut des Benutzers ist; und
ein Modul zur Erfassung elektrischer Signale, wobei das Modul zur Erfassung elektrischer Signale biometrische elektrische Signale von dem Benutzer durch den Kontakt der Elektrode mit der Haut des Benutzers erfasst.

2. Elektrodentestvorrichtung (100) gemäß Anspruch 1, wobei die mindestens eine Benutzerbefestigung (201) Führungen zum Halten der Gliedmaße oder des Fingers des Benutzers beinhaltet.

3. Elektrodentestvorrichtung (100) gemäß Anspruch 1 oder Anspruch 2, wobei die mindestens eine Benutzerbefestigung (201) verstellbare Spannungsstifte (202) beinhaltet, wobei optional die verstellbaren Spannungsstifte (202) konfiguriert sind, um übereinstimmende inkrementelle Spannungswerte an mindestens einer Benutzerbefestigung (201) bereitzustellen.

4. Elektrodentestvorrichtung (100) gemäß einem der vorhergehenden Ansprüche, wobei die mindestens eine Elektrode ein trockenes Material ist.

5. Elektrodentestvorrichtung (100) gemäß einem der vorhergehenden Ansprüche, wobei die Elektrodenbefestigungshalter (103) von der Elektrodentestvorrichtung (100) abnehmbar sind, wobei optional die Vielzahl von Elektrodenmaterialprobenhaltern eine Vielzahl von Materialkontaktbereichen beinhaltet.

6. Elektrodentestvorrichtung (100) gemäß einem der vorhergehenden Ansprüche, wobei die mindestens eine Benutzerbefestigung (201) von der Elektrodentestvorrichtung (100) abnehmbar ist, wobei optional eine der mindestens einen der Benutzerbefestigungen (201) durch eine Elektrode vom Gurtbandtyp ersetzt ist, die dazu geeignet ist, mittels eines Gurtbands am Körper eines Benutzers angebracht zu werden.

7. Elektrodentestvorrichtung (100) gemäß einem der vorhergehenden Ansprüche, die ferner eine Energiequelle (101) beinhaltet, wobei die Energiequelle (101) eine Batterie ist.

8. Elektrodentestvorrichtung (100) gemäß einem der vorhergehenden Ansprüche, die ferner einen Anzeigebildschirm (105) beinhaltet, wobei der Anzeigebildschirm (105) ein LCD-Bildschirm ist, wobei optional der Anzeigebildschirm (105) Informationen in Bezug auf den Detektionsstatus, den Verbindungsstatus, die Signalqualität, biometrische elektrische Signalwerte, die Elektrodentemperatur und/oder die Batterielebensdauer anzeigt.

9. Elektrodentestvorrichtung (100) gemäß einem der vorhergehenden Ansprüche, die ferner ein Drahtlosnetzwerkkommunikationsmodul beinhaltet.

10. Elektrodentestvorrichtung (100) gemäß einem der Ansprüche 1 bis 9, wobei die biometrischen elektrischen Signale konfiguriert sind, um das Hochladen in eine Cloud zu ermöglichen, und unter Verwendung von Software zur biometrischen Analyse aus der Ferne analysiert werden.

11. Ein Elektrodentestverfahren der Elektrodentestvorrichtung (100) gemäß einem der vorhergehenden Ansprüche, wobei das Elektrodentestverfahren Folgendes beinhaltet:
Installieren einer oder mehrerer Elektroden in dem mindestens einen Elektrodenbefestigungshalter (103);
Platzieren einer Gliedmaße oder eines Fingers eines Benutzers in der mindestens einen Benutzerbefestigung (201); und
Erfassen eines biometrischen elektrischen Signals von dem Benutzer von dem Modul zur Erfassung elektrischer Signale.

12. Elektrodentestverfahren gemäß Anspruch 11, wobei das biometrische elektrische Signal ein Elektrokardiogrammsignal, EKG-Signal, ist.

13. Elektrodentestverfahren gemäß Anspruch 11, das ferner das Erfassen von Offsetpotentialdaten jeder Elektrodenschnittstelle beinhaltet.

14. Ein System (500) zum Testen von Elektroden, das Folgendes beinhaltet:
eine Elektrodentestvorrichtung (100) gemäß einem der Ansprüche 1-10;
eine elektronische Rechenvorrichtung (502), die Software (504) zur biometrischen Analyse beinhaltet;
ein Drahtloskommunikationssystem, das betreibbar ist, um die Kommunikation von Daten zwischen der Elektrodentestvorrichtung (100) und der elektronischen Rechenvorrichtung (502) zu bewirken; und
einen Cloud-Server (503).

15. System (500) gemäß Anspruch 14, das ferner einen digitalen Temperaturmesser beinhaltet.

## Revendications

1. Un dispositif de test d'électrodes (100), pour tester des électrodes de détection biométriques, le dispositif de test d'électrodes (100) comprenant :
au moins un support de montage d'électrode (103), destiné à recevoir une électrode à tester, dans lequel l'au moins un support de montage d'électrode (103) comprend une fente de montage de matériau pour électrode (203), une pluralité de supports d'échantillons de matériau pour électrode et au moins une monture pour utilisateur (201) destinée à recevoir un membre ou un doigt d'un utilisateur, dans lequel un support d'échantillon de matériau pour électrode (204) de la pluralité de supports d'échantillons de matériau pour électrode est accueilli à l'intérieur de la fente de montage de matériau pour électrode (203), dans lequel l'électrode à tester est placée dans le support d'échantillon de matériau pour électrode de la pluralité de supports d'échantillons de matériau pour électrode, et dans lequel l'au moins une monture pour utilisateur (201) est configurée pour permettre à l'électrode d'être en contact avec la peau de l'utilisateur ; et
un module d'acquisition de signal électrique, dans lequel le module d'acquisition de signal électrique acquiert des signaux électriques biométriques provenant de l'utilisateur par le biais du contact de l'électrode avec la peau de l'utilisateur.

2. Un dispositif de test d'électrodes (100) de la revendication 1 dans lequel l'au moins une monture pour utilisateur (201) comprend des guides destinés à recevoir le membre ou le doigt de l'utilisateur.

3. Un dispositif de test d'électrodes (100) de la revendication 1 ou de la revendication 2 dans lequel l'au moins une monture pour utilisateur (201) comprend des goupilles de tension variable (202), facultativement dans lequel les goupilles de tension variable (202) sont configurées pour fournir des valeurs de tension incrémentielle correspondantes sur au moins une monture pour utilisateur (201).

4. Le dispositif de test d'électrodes (100) de n'importe quelle revendication précédente, dans lequel l'au moins une électrode est un matériau sec.

5. Le dispositif de test d'électrodes (100) de n'importe quelle revendication précédente, dans lequel les supports de montage d'électrode (103) sont détachables du dispositif de test d'électrodes (100), facultativement dans lequel la pluralité de supports d'échantillons de matériau pour électrode comprennent une pluralité de zones de contact avec le matériau.

6. Le dispositif de test d'électrodes (100) de n'importe quelle revendication précédente, dans lequel l'au moins une monture pour utilisateur (201) est détachable du dispositif de test d'électrodes (100), facultativement dans lequel une de ces au moins une des montures pour utilisateur (201) est remplacée par une électrode de type à sangle appropriée pour être maintenue sur le corps d'un utilisateur au moyen d'une sangle.

7. Le dispositif de test d'électrodes (100) de n'importe quelle revendication précédente, comprenant en outre une source d'alimentation (101), dans lequel la source d'alimentation (101) est une batterie.

8. Le dispositif de test d'électrodes (100) de n'importe quelle revendication précédente, comprenant en outre un écran d'affichage (105), dans lequel l'écran d'affichage (105) est un écran LCD, facultativement dans lequel l'écran d'affichage (105) affiche des informations relatives à un état de détection, un état de connexion, une qualité de signal, des valeurs de signaux électriques biométriques, une température d'électrode, et/ou une durée de vie de batterie.

9. Le dispositif de test d'électrodes (100) de n'importe quelle revendication précédente, comprenant en outre un module de communication de réseau sans fil.

10. Le dispositif de test d'électrodes (100) de n'importe lesquelles des revendications 1 à 9, dans lequel les signaux électriques biométriques sont configurés pour permettre un téléchargement vers un nuage et sont analysés à distance en utilisant un logiciel d'analyse biométrique.

11. Un procédé de test d'électrodes du dispositif de test d'électrodes (100) selon n'importe quelle revendication précédente, le procédé de test d'électrodes comprenant :
installer une ou plusieurs électrodes dans l'au moins un support de montage d'électrode (103) ;
placer un membre ou un doigt d'un utilisateur dans l'au moins une monture pour utilisateur (201) ; et
acquérir, à partir du module d'acquisition de signaux électriques, un signal électrique biométrique provenant de l'utilisateur.

12. Le procédé de test d'électrodes de la revendication 11, dans lequel le signal électrique biométrique est un signal d'électrocardiographe, ECG.

13. Le procédé de test d'électrodes de la revendication 11, comprenant en outre acquérir des données de potentiel de décalage de chaque interface d'électrode.

14. Un système (500) pour tester des électrodes, comprenant :
un dispositif de test d'électrodes (100) selon n'importe lesquelles des revendications 1 à 10 ;
un dispositif informatique électronique (502) comprenant un logiciel d'analyse biométrique (504) ;
un système de communication sans fil utilisable pour effectuer une communication de données entre le dispositif de test d'électrodes (100) et le dispositif informatique électronique (502) ; et
un serveur en nuage (503).

15. Le système (500) de la revendication 14, comprenant en outre un thermomètre numérique.
